# EUROPEAN PATENT APPLICATION

(11) **EP 4 378 475 A1**
(43) Date of publication of application: **05.06.2024**
(21) Application number: 22757824.2
(22) Date of filing: 21.07.2022
(51) Int. Cl.: A61K 39/12, A61P 31/14, C07K 19/00

(54) **RECOMBINANT ANTIGEN FOR INDUCING AN IMMUNE RESPONSE AGAINST THE ZIKA VIRUS**

(30) Priority: 28.07.2021 CU 20210063
(71) Applicant: Centro de Ingeniería Genética y Biotecnología, La Habana 11600 (CU)
(72) Inventor: VALDÉS PRADO, Iris, La Habana 11600 (CU); GIL GONZALEZ, Lázaro, La Habana 11600 (CU); LAZO VÁZQUEZ, Laura, La Habana 17100 (CU); HERMIDA CRUZ, Lisset, La Habana 17100 (CU); GUILLEN NIETO, Gerardo, Enrique, La Habana 11600 (CU); COBAS ACOSTA, Karem, La Habana 17100 (CU); ROMERO FERNÁNDEZ, Yaremis, La Habana 17100 (CU); BRUNO DARDER, Andy, Jesús, La Habana 11000 (CU); SUZARTE PORTAL, Edith, La Habana 13800 (CU); PÉREZ FUENTES, Yusleidi, de la Caridad, La Habana 10700 (CU)
(74) Representative: Capitán García, Maria Nuria
(86) International application number: PCT/CU2022/050008
(87) International publication number: WO 2023/006131

(57) **Abstract**

The invention relates to a recombinant chimeric antigen comprising, in the polypeptide chain thereof, a polypeptide corresponding to amino acids 2 to 104 of the Zika virus capsid protein or a polypeptide with an amino acid sequence with at least 90% identity with said region of the capsid protein. The invention also relates to a vaccine composition comprising said recombinant chimeric antigen and a pharmaceutically acceptable vaccine adjuvant. The invention further relates to the use of the recombinant chimeric antigen, comprising, in the polypeptide chain thereof, a polypeptide corresponding to amino acids 2 to 104 of the Zika virus capsid protein or an amino acid sequence with at least 90% identity with said segment for the production of a drug for inducing an immune response against the Zika virus. The invention also discloses a method for inducing an immune response against the Zika virus, in which the recombinant chimeric antigen is administered.

## Description

### Technique field

The present invention relates to the field of biotechnology and pharmaceutical industry, in particular with the obtaining of recombinant protein antigens and vaccine compositions against zika virus.

### Background of the previous technique

Zika virus belongs to flavivirus genus, of the *Flaviviridae family* (Kuno & Chang, 2007, Arch Virol, 152:687-696), which includes other pathogens like Yellow fever virus, Japanese encephalitis virus, West Nile virus, Tick-borne encephalitis virus and dengue virus. Zika virus is transmitted to humans through the bite of infected mosquito of *Aedes* genus (Kindhauser et al., 2016, Bull World Health Organ, 94:675-686C), which is present in abundance, in tropical and subtropical regions (Sharma & Lal, 2017, Front Microbiol, 8:110-). Zika is an enveloped virus with a positive single-strand RNA, which is translated to a polyprotein, which is co- and post-translationally cleaved into ten individual proteins (Kuno & Chang, 2007, Arch Virol, 152:687-696): three structural proteins and seven nonstructural proteins, which are involved in viral replication, virus assembly, and evasion of the immune system (Sirohi et al., 2016, Science, 352:467-470). The structural proteins are: capsid protein (C), envelope protein (E) and the membrane protein (M) or the membrane precursor protein (PrM)). Nonstructural proteins are known as NS1, NS2a, NS2b, NS3, NS4a, NS4b, and NS5.

So far, there have been three large-scale zika virus outbreaks, the first one occurred in the Federal State of Micronesia in 2007, with about 7,000 people infected, but without serious complications (Duffy et al., 2009, N Engl J Med, 360:2536-2543). In 2013, the second epidemic outbreak occurred in French Polynesia, where an estimated of 28,000 inhabitants suffered from zika virus infection (Musso et al., 2014, Clin Microbiol Infect, 20:O595-O596). In this outbreak, an increase in the incidence of cases of neurological damage such as Guillain-Barré Syndrome was observed for the first time (Cao-Lormeau et al., 2016, Lancet, 387:1531-1539). The third outbreak was in 2015, in Brazil, with 440,000 - 1,300,000 suspected cases of zika virus infection (Hennessey et al., 2016, MMWR Morb Mortal Wkly Rep, 65:55-58). In that outbreak, there was an increase in the number of cases with neurological damage, specifically persons with Guillain-Barré Syndrome and newborns with microcephaly (Schuler-Faccini et al., 2016, MMWR Morb Mortal Wkly Rep, 65:59-62).

In 2016, the World Health Organization (WHO) declared the disease caused by the zika virus a "Public Health Emergency of International Concern". In 2017, the transmission of the disease was reported in 48 countries, which accumulated 510,000 suspected cases and more than 170,000 confirmed cases (Yun & Lee, 2017, J Microbiol, 55:204-219). As of 2019, 87 countries distributed in four of the six WHO regions (Africa, Southeast Asia, the Western Pacific, and the Americas) had reported autochthonous transmission of zika virus through infected mosquito bites. Specifically for this virus, in addition to vector transmission, other routes of transmission such as sexual transmission, mother to fetuses during pregnancy, and blood transfusions are possible (Frank et al., 2016, Euro Surveill, 21).

After the infection, the clinical manifestation a "dengue-like syndrome" is developed in humans, which can be mild or asymptomatic, approximately 80% of infections are asymptomatic, (Grossi-Soyster & LaBeaud, 2017, Curr Opin Pediatr, 29:102-106). In general, symptomatic cases are mild and may include rash, febrile state, arthritis, arthralgia, non-purulent conjunctivitis and edema of the extremities (Calvet et al., 2016, Curr Opin Infect Dis, 29:459-466). Other symptoms may also occur, such as: headache, myalgia, retro-orbital pain, lower back pain, lymphadenopathy, and vomiting (Brasil et al., 2016, PLoS Negl Trop Dis, 10:e0004636). The disease is often mild; however there are reports of fatal cases, particularly in persons with underlying medical conditions (Arzuza-Ortega et al., 2016, Emerg Infect Dis, 22:925-927).

Early data indicate that the immune response to zika virus is similar to that of other flaviviruses, where neutralizing antibodies against the E protein (Fernandez & Diamond, 2017, Curr Opin Virol, 23:59-67), constitute the main element in protection against infection (Sapparapu et al., 2016, Nature, 540:443-447). The E protein is the most exposed on the virion surface, and it is involved in the fusion and entry of the virus into the host cell (Larocca et al., 2016, Nature, 536:474-478). However, the high level of cross-reactivity between flaviviruses, especially between dengue viruses and zika virus, hinder the role of antibodies to discriminate between both viral infections (Kostyuchenko et al., 2016, Nature, 533:425-428).

So far, it is uncertain whether the antibodies generated against the zika virus could facilitate the viral infection to another related flavivirus through the phenomenon of Antibody-Dependent Enhancement of infection, as has been well documented for dengue viruses. It is also not known whether the immune response generated against zika virus could affect the subsequent infection with dengue viruses or vice versa, or whether neurological damage, such as Guillain-Barré syndrome observed in some patients, is a consequence of the direct viral infection or secondary effects of the immune response (Poland et al., 2018, Lancet Infect Dis, 18:e211-e219).

On the other hand, recent studies indicate an important role for the T cell response against zika virus infection. Using peripheral blood mononuclear cells (PBMC) from people immune to the virus, the main targets of the cellular immune response have been identified. It is primarily directed against structural proteins C and prM, and nonstructural proteins NS2 and NS5 (Xu et al., 2016, PLoS Curr, 8:, Elong et al., 2017, Cell Host Microbe, 21 :35-46).

More than 40 vaccine candidates against zika virus are currently in development, using multiple antigen-delivery approaches, including inactivated virus vaccines, attenuated vaccines, DNA and mRNA vaccines, viral vector-based vaccines and subunit vaccines (Barouch et al., 2017, Immunity, 46:176-182). However, some of these approaches have disadvantages such as its high production costs and low safety profiles, opening the way for subunit vaccines.

Among subunit vaccines, there are variants based on the 80% of the E protein (Liang et al., 2018, PLoS One, 13:e0194860-; Qu et al., 2018, Antiviral Res, 154:97-103), and on the domain III of the E protein (EDIII) (Qu et al., 2018, Antiviral Res, 154:97-103). Specifically, the EDIII region emerges as a promising immunogen, based on the results obtained for other flaviviruses (Beltramello et al., 2010, Cell Host Microbe, 8:271-283). Several studies using the recombinant EDIII region have shown the induction of humoral and cellular immune responses, as well as protection, against viral challenge in mice (Yang et al., 2017, Sci Rep, 7:7679-; Qu et al., 2018, Antiviral Res, 154:97-103). However, not all of these strategies have been able to reproduce these promising results in animal models closer to humans. To improve the humoral and cellular-induced immune responses new formulations have been evaluated including new antigens, improving the presentation to the immune system or combining recombinant subunits with adjuvants.

According to the elements mentioned above, the development of subunit vaccines against zika virus, capable of inducing a safe and effective immune response against this flavivirus, is still an unsolved problem.

### Description of the invention

The present invention solves the aforementioned problem by providing recombinant chimeric antigens that comprise in their polypeptide chain, a polypeptide corresponding to amino acids 2 to 104 of the zika virus capsid protein or a polypeptide with an amino acid sequence with at least 90% identity with such a region of the capsid protein of that virus. In this way, the chimeric antigens of the invention contain regions that potentially induce cellular immune response, capable of reducing the viral load and providing protection, without the presence of neutralizing antibodies to the virus.

In one embodiment of the invention, the chimeric antigens additionally comprise a polypeptide corresponding to the EDIII domain of the zika virus E protein or a polypeptide with an amino acid sequence with at least 90% identity with such domain of the zika virus envelope protein. In this case, the chimeric antigens comprise two potentially protective regions, capable of inducing both neutralizing antibodies and a cellular immune response. These regions consist of 145 amino acids from the EDIII domain and 103 amino acids from the viral capsid protein. Both viral regions present a high degree of homology between the different isolates, which comprise the two lineages that have been identified for the zika virus, the Asian/American lineage and the African lineage (Haddow et al., 2012, PLoS Negl Trop Dis, 6:e1477-). These regions convert the chimeric antigens of the invention into useful molecules to protect against infection by different strains of the zika virus.

In one embodiment of the invention, chimeric antigens additionally comprise a segment of six histidine residues at the N-terminus of their polypeptide chain. In a particular embodiment, the chimeric antigen has an amino acid sequence that is identified as SEQ ID NO: 9 or SEQ ID NO: 10.

The object of the invention is a vaccine composition that comprises the recombinant chimeric antigen that comprises in its polypeptide chain a) a polypeptide corresponding to amino acids 2 to 104 of the zika virus capsid protein or a polypeptide with an amino acid sequence with at least 90% identity with such region of the capsid protein of that virus and b) a pharmaceutically acceptable vaccine adjuvant. In one embodiment of the invention the adjuvant is an aluminum salt.

The ability of the vaccine compositions to induce immunity was firstly evaluated in preclinical studies in immunocompetent mice (BALB/c), which were immunized by intraperitoneal route. In these studies, alum was used as adjuvant, due to its versatility for later use in humans. In addition, the vaccine compositions were also tested in combination with an immune-stimulatory deoxyribonucleic acid (sDNA) chain (referred to as SEQ ID NO: 11 in the present invention). This allowed the generation of new vaccine compositions where their immunogenicity is increased, when compared to preparations without the immune-enhancer, due to the formation of aggregates of these antigens, which are more efficiently presented to the immune system than nonaggregated variants.

Therefore, in an embodiment of the invention, the vaccine composition based on chimeric antigens additionally comprises a nucleic acid that has a nucleotides sequence that is identified as SEQ ID NO: 11. In one embodiment of the invention, in the vaccine composition the chimeric antigen is in a range of 10-150 micrograms per dose.

Vaccine compositions including the viral capsid region, both soluble and in aggregate form due to the presence of the immune-stimulatory ssDNA, generated a cellular immune response in immunized mice. This immunity conferred partial protection to the animals after the challenge with different virus strains.

In the case of vaccine compositions including the chimeric protein comprising the EDIII region of zika virus and the viral capsid protein region, in soluble form and in aggregated form after combination with the immune-stimulatory ssDNA, they were able to generate a humoral immune response in immunized mice, as well as an effective cellular immune response. This immunity conferred protection to the animals after the challenge with different strains of the virus.

Subsequently, the vaccine compositions that include the aggregated variants of both molecules were chosen and evaluated in non-human primates of the Macaca *mulatta* species, which were inoculated subcutaneously, also using alum as adjuvant. As a result, neutralizing antibodies against the zika virus were generated in immunized animals, as well as a cellular immune response after three doses. Therefore, in another aspect, the invention comprises the use of the recombinant chimeric antigens described above for the manufacture of a drug for the induction of an immune response against the zika virus.

Also, the invention provides a method for inducing an immune response against the zika virus in a person where a pharmaceutically effective amount of the recombinant chimeric antigen that comprises, in its polypeptide chain, a polypeptide corresponding to amino acids 2 to 104 of the zika capsid protein or a polypeptide having an amino acid sequence with at least 90% identity with such region of the zika virus capsid protein and a pharmaceutically acceptable adjuvant, is administered.

In one embodiment of the invention, the adjuvant is an aluminum salt. In one embodiment of the method of the invention, the pharmaceutically effective amount of the recombinant chimeric antigen is in a range of 10-150 micrograms per dose. As a particular embodiment, in the method of the invention, a nucleic acid that has a nucleotide sequence identified as SEQ ID NO: 11 is additionally administered together with the recombinant chimeric antigen.

Although several studies using the EDIII region of the zika virus have shown the induction of humoral and cellular immune responses, and protection against viral challenge in mice, these strategies have not been able to reproduce the good immunogenicity results achieved in primates. The present invention has obvious advantages compared to those attempts to develop vaccine candidates based on zika virus subunits. The chimeric antigens of the invention, in vaccine compositions containing them, have been shown to induce both cellular and humoral responses in non-human primates. In part, this effective response against zika virus is due to the inclusion of a 103 amino acid fragment of the virus capsid protein, and to a better way of presenting the antigen to the immune system.

### Brief description of the figures

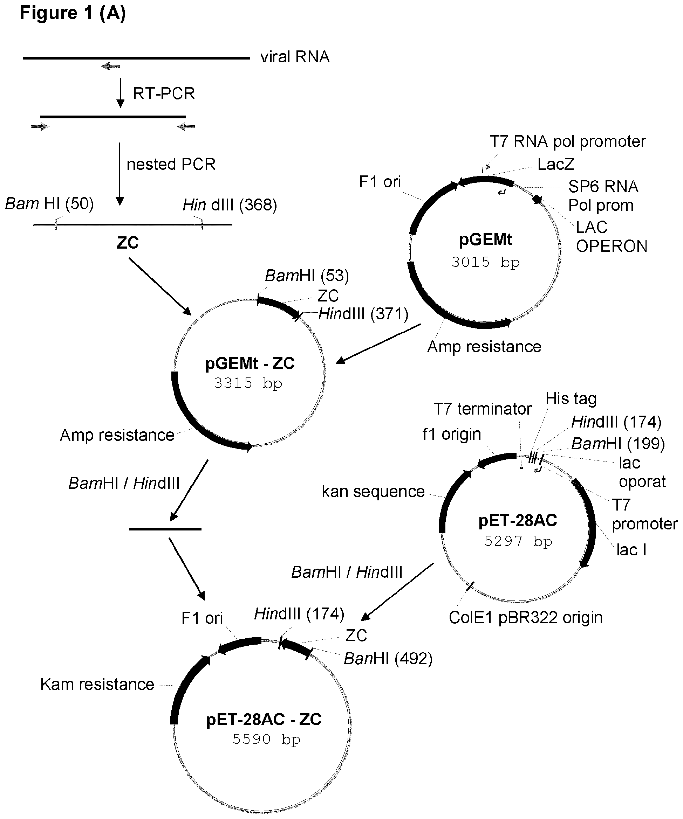

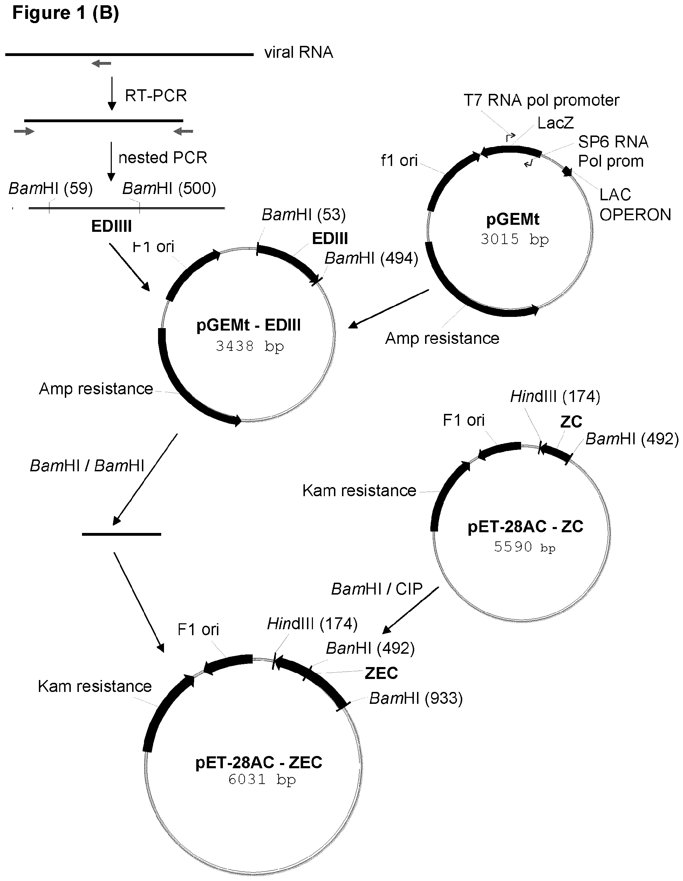

**Figure 1****.** Cloning of the encoding regions for the ZC **(A)** and ZEC **(B)** proteins in the expression vector pET-28AC.

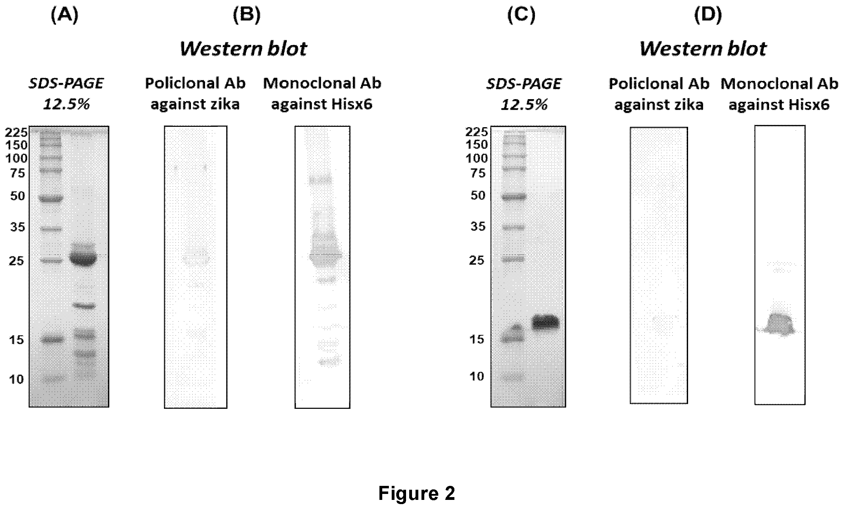

**Figure 2****.** Separation by SDS polyacrylamide gel electrophoresis (SDS-PAGE) and Western blot characterization of ZEC and ZC recombinant antigens: **(A)** 12.5% SDS-PAGE of ZEC protein; **(B)** Western blot of ZEC protein; **(C)** 12.5% SDS-PAGE of ZC protein and **(D)** Western blot of ZC protein.

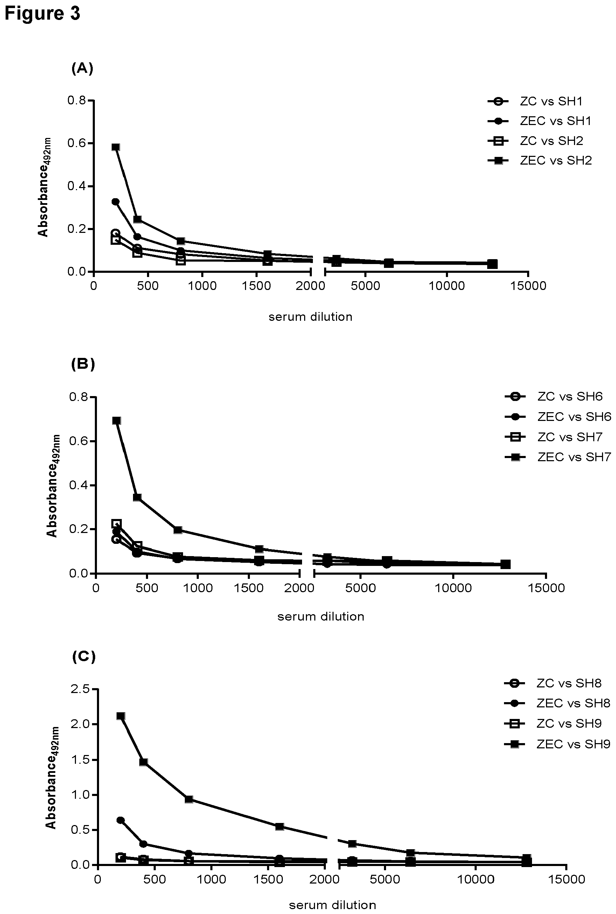

**Figure 3****.** Antigenic characterization by ELISA of ZC and ZEC recombinant proteins using polyclonal antibodies from zika-immune subjects. The figure shows the reactivity of the two recombinant proteins with serial dilutions of human sera SH1 and SH2 **(A),** SH6 and SH7 **(B),** SH8 and SH9 **(C).**

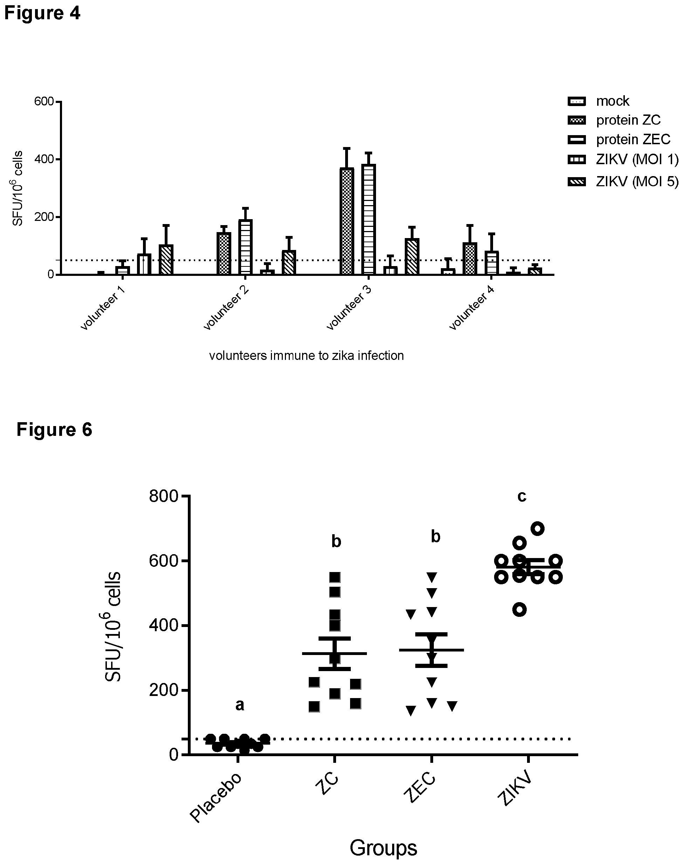

**Figure 4****.** Interferon gamma (IFNγ) ELISpot using PBMC from zika-immune people after the *in vitro* stimulation with the recombinant proteins or the zika virus. 10 µg of ZC or ZEC recombinant protein was used to the *in vitro* stimulation, and a multiplicity of infection (MOI) of 1 or 5 was used to the *in vitro* stimulation with virus. Results are shown as mean ± standard deviation of spot formation units (SFU) per one million of PBMC, from two independent experiments. The dashed line indicates the positivity limit of the assay (50 SFU/million cells).

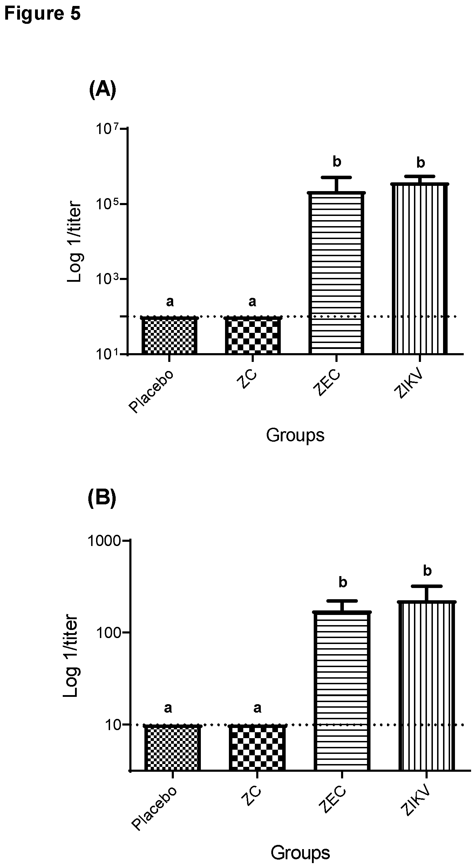

**Figure 5****.** Humoral immune response in BALB/c mice immunized with the compositions comprising the ZC or ZEC proteins. **(A)** Antibodies anti-zika virus determined by ELISA. **(B)** Neutralizing antibodies measured by the *in vitro* viral neutralization test. Data are presented as mean ± standard deviation (n=10). Different letters indicate statistically significant differences among the groups (*p*<0.01). The dashed horizontal line indicates the positivity limit of the assay.
Figure 6. Cell-mediated immune response in BALB/c mice immunized with the ZC or ZEC proteins, determined by IFNγ ELISpot. Data are presented as the mean ± standard error of the mean of SFUs per million spleen cells (n=10). Different letters indicate statistically significant differences among the groups (*p*<0.001). The dashed horizontal line indicates the positivity limit of the assay.

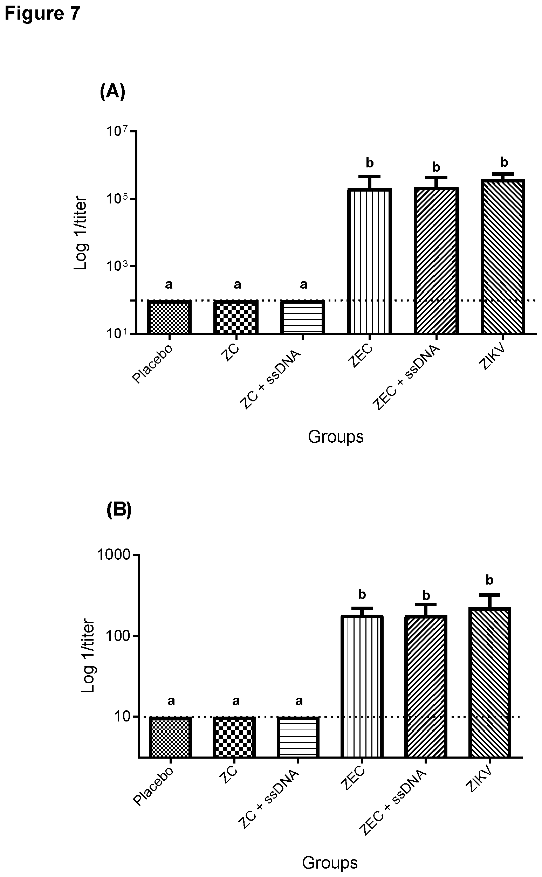

**Figure 7****.** Humoral immune response in BALB/c mice immunized with the ZC or ZEC protein formulations. **(A)** Zika virus antibodies measured by ELISA. **(B)** Neutralizing antibodies determined by the *in vitro* viral neutralization test. Data are presented as mean ± standard deviation (n=10). Different letters indicate statistically significant differences among the groups (*p*<0.01). The dashed horizontal line indicates the positivity limit of the assay.

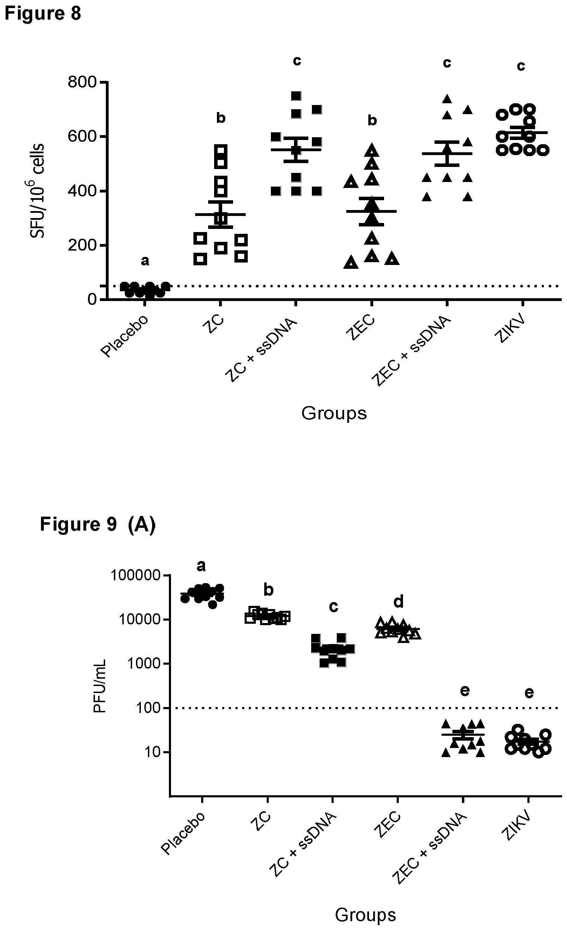

**Figure 8****.** Cell-mediated immune response in BALB/c mice immunized with ZC or ZEC proteins, determined by IFNγ ELISpot. Data are presented as the mean ± standard error of SFU per million spleen cells (n=10). Different letters indicate statistically significant differences among the groups (*p*<0.01). The dashed horizontal line indicates the positivity limit of the assay.

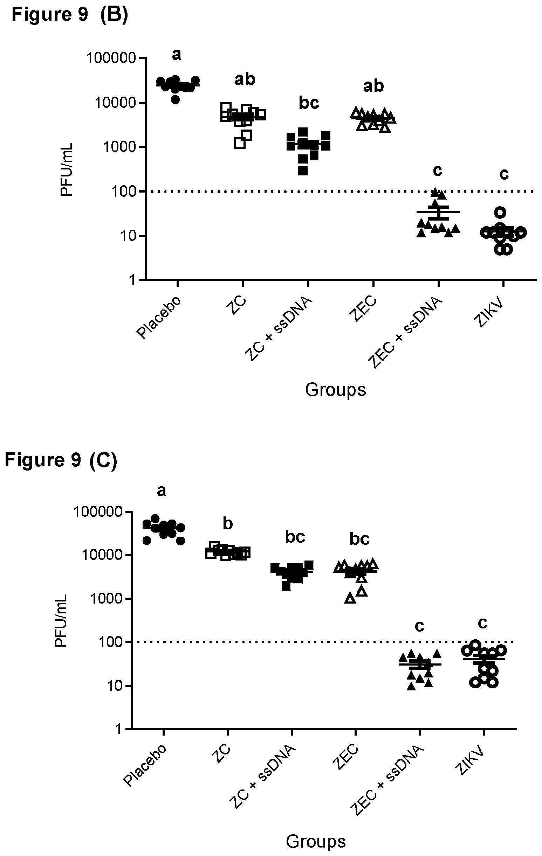

**Figure 9****.** Viremia in the brain of BALB/c mice immunized with recombinant proteins ZC or ZEC and challenged with different neuroadapted strains of zika virus. **(A)** Protection assay after the viral challenge with a strain isolated from a Cuban patient (ZIK16 strain); **(B)** viral challenge with the reference strain MR766; **(C)** Protection assay after the viral challenge with the reference strain Brazil ZKV2015. Data are presented as the mean ± standard error of the mean (n=10). The dashed line represents the detection limit of the assay. Different letters indicate statistically significant differences among the groups (*p*<0.05).

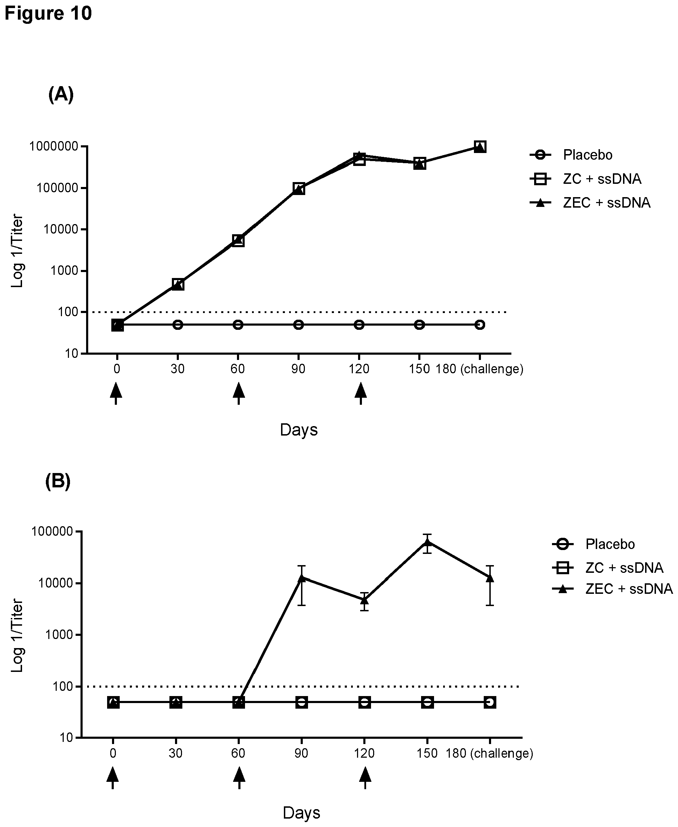

**Figure 10****.** Kinetics of the humoral immune response in non-human primates immunized with the compositions ZEC+DNAss or ZC+DNAss. **(A)** Anti-recombinant protein antibodies (ZC or ZEC) measured by ELISA. **(B)** Antibodies against zika virus measured by ELISA. The arrows indicate the administration times of each dose. The dashed horizontal line indicates the positivity limit of the assay.

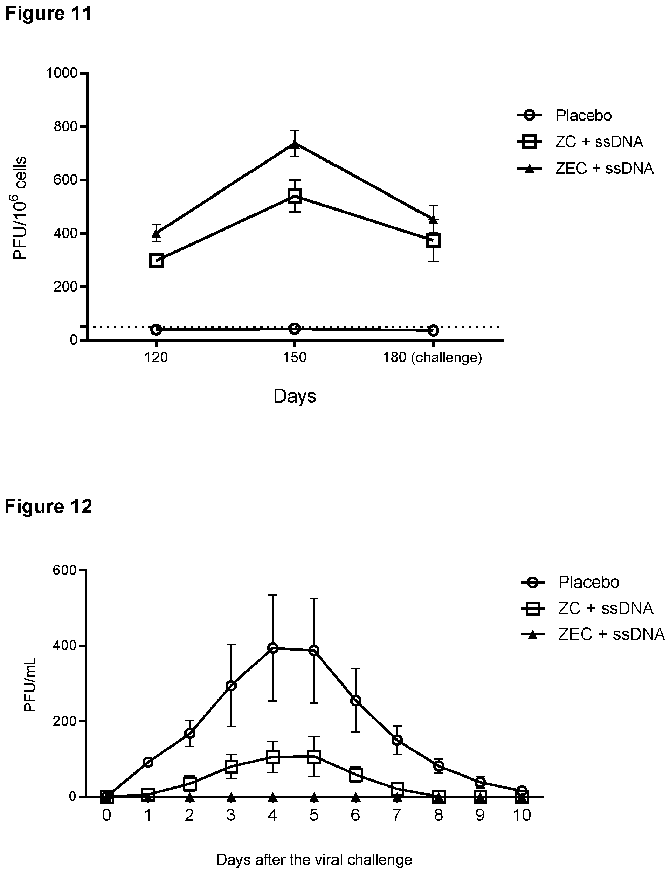

**Figure 11****.** Kinetics of the cell-mediated immune response determined by IFNγ ELISpot in non-human primates immunized with the compositions ZEC+DNAss or ZC+DNAss. Data are presented as the mean ± standard error of the mean of SFU per million PBMC (n=4). The dashed horizontal line indicates the positivity limit of the assay.
**Figure 12****.** Viremia in non-human primates immunized with the compositions ZC+DNAss or ZEC+DNAss and challenged with infective zika virus (ZIK16 strain). Data are presented as the mean ± standard deviation (n=4).

### Detailed exposition of embodiments / Examples of embodiment

### Example 1. Cloning and expression of the recombinant proteins ZC and ZEC.

Vero cells were infected with urine and serum samples of volunteers with symptomatic and confirmed ZIKV infection, collected between day 3 and day 7 after the onset of the symptoms of the disease. After 6-7 days of incubation, successive passages in Vero cells were made, and the culture supernatants were collected when cytopathic effect (syncytial formation) was observed. After 7-8 successive passages, preparations were titrated on Vero cells and viral titers of 7×10⁶ PFU/mL were obtained. Similar results have been described in isolation experiments for zika virus from infected people's samples (Musso et al., 2015, J Clin Virol, 68:53-55; Bonaldo et al., 2016, PLoS Negl TropDis, 10:e0004816).

The RNA was extracted from the culture supernatants of Vero cells infected and was used as a template in the reverse transcription reaction for the amplification of the DNA chain encoding for the virus capsid and envelope proteins. As primers, oligonucleotides that bind to flanked conserved regions near both viral proteins were used, which are shown in Table 1.

**Table 1. Sequence of the DNA oligonucleotides used for the isolation of the encoding regions for the viral capsid and envelope proteins.**

| **SEQ ID NO:** | **Name** | **Sequence (5'- 3')** |
|---|---|---|
| 1 | *sense* E zika | CATGATACTGCTGATTGCCCCG |
| 2 | *antisense* E zika | GAAGTCCACTGAGCACCCCACG |
| 3 | *sense* ZC zika | ATTTGGAAACGAGAGTTTCTGGTCATG |
| 4 | *antisense* ZC zika | CGGCATCGCTCCTATCCAAGTACA |

DNA sequences were amplified by the polymerase chain reaction (PCR), both PCR fragments were then sequenced and were compared with those previously described for other zika virus isolates, demonstrating the identity of the regions obtained. Zika virus isolated from the Cuban patient's sample was referred to as ZIK16 from now on. Subsequently, a second PCR was performed using these regions as a template for the amplification of the regions corresponding to ZC and the EDIII region, with the specific restriction sites for subsequent cloning steps (Table 2).

**Table 2. DNA sequences of the oligonucleotides used for the amplification of the encoding regions for the ZC protein and the EDIII domain, with the restriction sites for cloning in the expression vector.**

| **SEQ ID NO:** | **Name** | **Sequence (5'- 3')*** |
|---|---|---|
| 5 | *sense* EDIII zika | CACTTA**GGATCC**CGCCTGAAAATGG |
| 6 | *antisense* EDIII zika | GTT**GGATCC**GCCTCCAACTGATCC |
| 7 | *sense* ZC zika (2) | CGAGAGTTTCTG**GGATCC**AAAAACCC |
| 8 | *antisense* ZC zika (2) | ACTAGT**AAGCTT**TCATCGTCTCTTCTTCTCCTT |

| | | |
|---|---|---|
| * The restriction site included in the oligonucleotides is represented underlined and in bold. | | |

Both fragments were cloned into the commercially available vector pGEMt, and transformed into competent JM-109 strain of *Escherichia coli* cells. The transformed cells were grown in Luria Bertani (LB) medium supplemented with 50 µg/mL of antibiotic ampicillin during 10 h at 37°C. These transformed cells of *E*. *coli* were used to obtain and purify the DNA plasmid. The intermediate constructs pGEMt-ZC and pGEMt-EDIII were digested with the restriction enzymes BamHI/Hindlll or BamHI, respectively, to obtain the DNA fragments encoding the viral capsid and EDIII regions of the zika virus. Next, the DNA band corresponding to the viral capsid was inserted into the pET-28AC expression vector. This vector contains the Phage T7 promoter and a tail of six histidine residues at the N-terminal region. As result, four transformants with the pET-28AC-ZC construct (Figure 1A) were obtained. They were sequenced and the identity of the encoding sequence for the ZC protein (SEQ ID NO: 9) was confirmed. Then, one of the construction pET-28AC-ZC (clone 2) was selected. This clone was digested with the BamHI/CIP enzymes for the insertion of the EDIII DNA band, previously digested with the BamHI enzyme, to obtain the resulting expression vector pET-28AC-ZEC. This vector includes the region of the EDIII domain fused to the N-terminal of the viral capsid protein. As result, two transformants of the pET-28AC-ZEC construct were chosen (Figure 1B). The identity of the encoding sequence for the ZEC protein was confirmed by sequencing and is identified in the sequence list as SEQ ID NO: 10.

The expression of the ZC and ZEC chimeric recombinant proteins was carried out in the BL-21(DE3) strain of *E*. *coli,* which was transformed with the plasmids pET-28AC-ZC (clone 2) and pET-28AC-ZEC (clone 23), respectively. The expression of recombinant protein was carried out after the induction of the Phage T7 promoter, with the addition of 1 mM of IPTG. The cell biomasses were analyzed by SDS-PAGE, and the overexpression of ZC (SEQ ID NO: 9) and ZEC (SEQ ID NO: 10) recombinant proteins have 12.8 kDa and 28.5 kDa of molecular weight, respectively.

### Example 2. Purification and antigenic characterization of the recombinant proteins ZC and ZEC.

The cell biomasses, obtained from the transformation of the BL-21(DE3) strain of *E*. *coli* with the constructions pET-28AC-ZC (clone 2) and pET-28AC-ZEC (clone 23), were resuspended in buffer saline phosphate and lysed after three passes of ultrasound. After disruption, both recombinant proteins were mostly distributed in the insoluble fractions obtained by centrifugation. From the insoluble fraction of each biomass, the recombinant proteins were solubilized in Tris buffer solution with 7 M Urea as chaotropic agent, and purified through an ion exchange chromatography process, using the SP *Sepharose FF* matrix. Each protein of interest was obtained by increasing the ionic strength of the buffer. Then, the Urea was removed and the proteins were refolded, through a sixe exclusion chromatography process in 10 mM Tris buffer pH 8.0. After both chromatographic processes, the ZC and ZEC proteins were obtained with 85% and 70% purity, respectively (Figures 2A and 2C).

The antigenic characterization of both proteins was performed by Western blot, using two different sources of antibodies (Figures 2B and 2D). The antibodies used were polyclonal antibodies from people immune to the virus and a monoclonal antibody that recognizes the tail of six histidine residues (MAb anti-His). It was confirmed that the ZEC protein is recognized by human polyclonal antibodies from people immune to zika virus infection. At the same time, this protein was immunoidentified with the anti-His MAb, which recognizes an epitope present at the N-terminal region of the ZEC protein. Likewise, smaller protein bands were also immunoidentified with this antibody, which indicates that during the purification of the ZEC protein, degradations occur in the C-terminal region.

In the Western blot characterization of the recombinant protein ZC, no recognition was observed by human polyclonal antibodies from people immune to virus infection, because the zika virus capsid protein is not exposed in the native viral particle. However, recognition was obtained with the anti-His MAb, which recognizes an epitope present at the N-terminal region of the recombinant antigen.

For primary structure verification of the of the recombinant proteins and the correct formation of the disulfide bond into the EDIII region in the ZEC chimeric polypeptide, the analysis of the recombinant proteins was carried out by mass spectrometry. The bands corresponding to each purified protein were separated by polyacrylamide gel electrophoresis in the presence of SDS and extracted from the gel, and then digested with the enzyme trypsin. The Table 3 summarizes the mass assignments of the spectra obtained, after digestion of both samples. Some of the signals were detected in both preparations, because they share the C-terminal of the molecule. After this analysis, it was possible to verify 62.7% and 41.7% of the ZEC (SEQ ID NO: 10) and ZC (SEQ ID NO: 9) protein sequence, respectively. In addition, the correct formation of the disulfide bond between the cysteine residues (Cys27 and Cys58), in the EDIII region found within the ZEC chimeric protein, was verified. This disulfide bond is essential for the proper conformation of the EDIII region, similar to how it is presented in the native structure of protein E (Sirohi et al., 2016, Science, 352:467-470; Sirohi & Kuhn, 2017 , J Infect Dis, 216:S935-S944). Surprisingly, the disulfide bond between the cysteine residues Cys27 and Cys58 was preserved, even when this region was fused to the viral capsid protein, to form the novel chimeric protein ZEC.

**Table 3. Mass/charge ratio values of the detected peptides and the sequence assignment.**

| **Sequence assignment^{a}** | **theoretical m/z** | **Charge** | **experimental m/z ZEC** | **experimental m/z ZC** |
|---|---|---|---|---|
| (²¹G-K³⁵)+(³⁶I-K⁵⁹) disulfide bond between Cys₂₇ y Cys₅₈ | 1020,50 | 4 | 1020,53 | - |
| | 1360,33 | 3 | 136,36 | - |
| ⁶⁰V-R⁷⁶ | 907,47 | 2 | 907,44 | - |
| | 605,32 | 3 | 605,30 | |
| ⁷⁷L-K⁹² | 858,95 | 2 | 858,92 | - |
| ⁹³M-K¹¹³ | 1155,57 | 2 | 1155,58 | - |
| ¹⁴⁰M-K¹⁵⁸ | 927,94 | 2 | 927,92 | - |
| ¹⁸⁰V-K¹⁸⁷ | 804,46 | 1 | 804,45 | 804,45 |
| ¹⁸⁹L-R²⁰¹ | 438,61 | 3 | 438,59 | 438,59 |
| | 657,41 | 2 | 657,38 | 657,38 |
| ²⁰²M-R²¹¹ | 573,86 | 2 | 573,85 | - |
| ²¹²F-R²²⁴ | 715,43 | 2 | 715,41 | 715,41 |
| | 477,29 | 3 | 477,28 | 477,26 |
| ²³²E-K²³⁸ | 417,23 | 2 | 417,21 | 417,20 |
| ²⁴³D-R²⁴⁹ | 789,43 | 1 | 789,42 | 789,40 |

| | | | | |
|---|---|---|---|---|
| ^{a} Number according to the sequence. | | | | |

In addition, the antigenic characterization of both recombinant proteins was carried out by ELISA using human sera. In the assay, the recombinant proteins ZC or ZEC were immobilized on the plate, and their recognition by human polyclonal antibodies was subsequently evaluated. For this assay, human sera (SH) from six volunteers immune to zika virus infection were used, which were collected during the acute phase (SH1, SH6 and SH8) or the convalescent phase of the disease (SH2, SH7 and SH9). As can be seen in Figure 3, there was greater recognition of the ZEC protein by polyclonal antibodies from people immune to the virus, in contrast to the low reactivity detected for the ZC protein. This behavior was similar for all the serum samples tested, which is due to the EDIII domain region included in the ZEC chimeric protein. This region is exposed on the surface of the virus particle (Sirohi et al., 2016, Science, 352:467-470; Sirohi & Kuhn, 2017, J Infect Dis, 216:S935-S944). Therefore, antibodies generated during natural infection recognize the ZEC protein. In contrast, the viral capsid protein is enveloped in the mature virion and antibodies to this region are not generated during zika virus infection (Mukhopadhyay et al., 2005, Nat Rev Microbiol, 3:13-22). This explains why the chimeric protein ZC, which only contains the capsid region, was not recognized by the sera of poeple who were infected with the zika virus.

Also, the recombinant proteins ZC and ZEC were evaluated for their capacity to stimulate the memory specific T cells to zika virus, using an IFNγ ELISpot assay. This cytokine have been described as a mediator of the cellular immune response through its antiviral activity against various flaviviruses (Shresta et al., 2004, J Virol, 78:2701-2710). For this experiment, PBMC from volunteers immune to the zika virus were used, which were *in vitro* stimulated with 10 µg/mL of the recombinant proteins ZC or ZEC, or with a preparation of zika virus (MOI of 1 and 5). Then, the number of memory T cells induced after stimulation, able of secreting the antiviral cytokine IFNγ, was quantified.

The results of that evaluation are shown in Figure 4, as the mean ± standard deviation of SFU per million cells, from two independent experiments. In this assay, zika virusspecific IFNγ-secreting cells were detected in samples from three of the four volunteers, after *in vitro* stimulation with the recombinant proteins ZC and ZEC. Likewise, after stimulation with the viral antigen, IFNγ-secreting cells specific for zika virus were detected in samples from three of the four volunteers analyzed. Taken together, these results demonstrate that the ZC and ZEC recombinant chimeric proteins include epitopes for memory T cells specific for zika virus. In turn, these protein antigens are able of stimulating the cellular immune response previously generated by the viral infection.

### Example 3. Evaluation of the immunogenicity and protective capacity of the recombinant proteins ZC and ZEC in BALB/c mice.

The evaluation of the immunogenicity of the recombinant proteins ZC and ZEC was carried out in female BALB/c mice, for which four groups of 10 animals each were used. The groups included in the study are shown in Table 4.

**Table 4. Design of the immunization scheme for the evaluation of the immunogenicity and protective capacity of the recombinant proteins ZC and ZEC in BALB/c mice.**

| **Group** | **Inmunogen** | **Adjuvant** | **Quantity of antigen** | **Volumen** |
|---|---|---|---|---|
| 1 | Placebo | Alum | - | 100 µL |
| 2 | Protein ZC | Alum | 10 µg | 100 µL |
| 3 | Protein ZEC | Alum | 20 µg | 100 µL |
| 4 | zika virus | - | 10⁵ PFU/mL | 250 µL |

For the animal's immunization, the recombinant proteins ZC or ZEC were used, taking into account their molecular weight, for that the group inoculated with the recombinant protein ZC received 10 µg and the group inoculated with the protein ZEC received 20 µg of recombinant protein. Both proteins were formulated in aluminum hydroxide (alum) as adjuvant at a final concentration of 1.44 mg/mL. All groups were immunized with three doses of each immunogen by intraperitoneal route on days 0, 15 and 45. Fifteen days after the last administration, the animals of each group were bled and the sera were used for the evaluation of the humoral immune response. Figure 5 shows the humoral immune response of antibodies against zika virus measured by ELISA and f neutralizing antibodies evaluated by the *in vitro* viral neutralization test.

For ELISA, plates were coated with zika virus (strain ZIK16), then serum samples were incubated at several dilutions, and then an appropriate dilution of commercial antimouse IgG conjugate was added. As seen in Figure 5A, high titers of antibodies against zika virus were detected in the groups immunized with the ZEC recombinant protein formulation and in the viral control group. There were no statistically significant differences between them (*p*>0.05). Statistical analysis was performed using Tukey's multiple comparison test. In the groups that received the Placebo and the recombinant protein ZC, no antibodies against the virus were detected, the levels detected were below the limit of positivity for the assay.

The functionality of the antibodies was measured through the *in vitro* plaque reduction viral neutralization test (PRNT) 30 days after the last dose. For the PRNT, dilutions of animal sera were mixed with a zika virus preparation (ZIK16 strain). After an incubation period, the mixtures were used to infect 24-well plates of Vero cells, thus the remaining not neutralized virus were able to infect the cells and cause the formation of visible plaques. Figure 5B shows the mean ± standard deviation of the neutralizing antibody titers against the zika virus, estimated in the sera of the immunized animals. The neutralizing titer was defined as the maximum dilution in which a 50% reduction in the number of plaques was achieved. In a similar way to the antibody response anti-zika virus detected, in the groups immunized with the protein ZEC formulation and the viral control group, neutralizing antibodies were detected, with Geometric Mean Titers (GMT) greater than 1:200, without statistical differences between them (*p*>0.05).

In addition, the study was evaluated the induction of the cellular immune response, 30 days after the last immunization in the animals immunized with the different formulations. For this assay, the spleen cells of the ten animals of each group were extracted, and the frequency of IFNγ-secreting cells was determined, after in vitro stimulation of the splenocytes with the zika virus. Figure 6 shows the number of cells secreting the antiviral cytokine in each group. In the groups immunized with the ZC and ZEC recombinant protein formulations, IFNγ-secreting cells were detected after *in vitro* stimulation of the splenocytes with the zika virus (ZIK16 strain), resulting in 100% of the responding animals in these groups. There were statistically significant differences among the values of IFNγ-secreting cells detected in the animals that received the ZC or ZEC recombinant proteins; and the number of IFNγ-secreting cells detected in the animals of the Placebo group (*p*<0.05). In the animals of the viral control group, the number of cells secreting the antiviral cytokine was higher compared to the levels of cells secreting IFNγ detected in the animals immunized with the recombinant proteins (*p*<0.05). Statistical analysis was performed using an ANOVA test and Tukey's multiple comparison.

### Example 4. Aggregation or formation of the nucleocapsid-like particles from the recombinant proteins ZC and ZEC.

The flavivirus viral capsid protein forms the nucleocapsid, which surrounds and protects the genome during the viral particle assembly process (Duffy et al., 2009, N Engl J Med, 360:2536-2543). Taking into account these structural characteristics, the recombinant proteins ZC and ZEC were used to carry out an in vitro aggregation process, or the formation of nucleocapsid-like particles (NSP), by incubating each recombinant protein with single-stranded DNA with immunomodulatory properties (herein called ssDNA, and identified as SEQ ID NO: 11).

Different combinations of protein and ssDNA were used in the aggregation reactions, according to the molecular weight of each molecule. Subsequently, the reaction mixtures were incubated for 30 minutes at 25°C, followed by 4 hours at 4°C, then the reaction mixtures were centrifuged at 10,000 × g, and the supernatants were analyzed by SDS-PAGE. As a result, it was observed that the soluble ZC or ZEC recombinant proteins could form insoluble high molecular weight aggregates at the several combinations evaluated, depending on the ssDNA quantity used in the aggregation reaction. For subsequent studies, an specific ratio of protein:ssDNA that favors the aggregation of approximately 50% of the recombinant protein of interest was chosen. The amounts of ssDNA required to achieve 50% aggregation were different for each recombinant protein, according to its mass and amino acid composition.

### Example 5. Evaluation of the immunogenicity of the compositions of the aggregated ZC and ZEC recombinant proteins.

For the evaluation of the immunogenicity of the recombinant aggregated proteins ZC and ZEC, six groups of female BALB/c mice were used, including 30 animals each. The groups included in the study are shown in Table 5.

**Table 5. Design of the immunization scheme for the immunolgical evaluation of the recombinant aggregated proteins ZC and ZEC.**

| **Group** | **Immunogen** | **Adjuvant** | **Quantity of antigen** | **Volumen** |
|---|---|---|---|---|
| 1 | Placebo (negative control) | Alum | - | 100 µL |
| 2 | ZC | Alum | 10 µg | 100 µL |
| 3 | ZC+ssDNA | Alum | 10 µg | 100 µL |
| 4 | ZEC | Alum | 20 µg | 100 µL |
| 5 | ZEC+ssDNA | Alum | 20 µg | 100 µL |
| 6 | zika virus | - | 10⁵ UFP/mL | 250 µL |

For the animal's immunization, the recombinant chimeric proteins ZC or ZEC, in soluble and aggregated forms after incubation with ssDNA, were used. Likewise, the molecular weight of each molecule was taken into account, for which the groups inoculated with the ZC recombinant protein received 10 µg of protein and the groups inoculated with the ZEC protein received 20 µg of recombinant protein. All formulations were prepared with alum as adjuvant, at a final concentration of 1.44 mg/mL. The groups received three doses of the formulations on days 0, 15 and 45 by intraperitoneal route.

Fifteen days after the last administration, 10 animals from each group were bled, and the serum was used to determine antibodies against zika virus by ELISA. As shown in Figure 7, high titers of antibodies against the virus were detected in the groups that received the formulations containing the chimeric protein ZEC, these titers were similar to those detected in the animals of the viral control group (*p*>0.05). This result indicates that the aggregation of the ZEC chimeric protein, due to the presence of ssDNA, did not affect the generation of antibodies against virus. In turn, in the animals immunized with the ZC and ZC+ssDNA formulations, no antibody titers against the zika virus were detected (Figure 7A). Statistical analysis was performed using a Kruskal-Wallis test and a *posteriori* multiple comparisons using Dunn's test.

The functionality of the antibodies elicited by the immunization was analyzed 30 days after the last dose by PRNT. Figure 7B shows the mean ± standard deviation of neutralizing antibody titers against zika virus. The neutralizing titer is defined as the maximum dilution in which a 50% reduction in the number of plaques is achieved. As can be seen, in the animals of the ZEC and ZEC+ssDNA groups, neutralizing antibodies were detected, with TPG greater than 1:200; which did not statistical differences with the titers detected in the group that received the zika virus preparation (*p*>0.05).

Additionally, in this immunization scheme, the capacity of the ZC and ZEC protein formulations to induce a cell-mediated immune response in immunized animals was evaluated. For that, 30 days after the last dose, spleen cells were extracted from ten animals of each group and the frequency of IFNγ-secreting cells was determined, after *in vitro* stimulation of splenocytes with zika virus. Figure 8 shows the number of secreting cells of the antiviral cytokine in each group. As can be seen, in the groups immunized with the ZC and ZEC recombinant protein formulations, 100% of the animals had IFNγ-secreting cells, with significant differences compared to the Placebo group (*p*<0.01). Statistical analysis was performed using an ANOVA test and Tukey's multiple comparison. Differences in the number of secretory cells were observed among the groups that received the soluble protein formulations and the aggregated formulations including ssDNA (*p*<0.01). In turn, the levels of IFNγ-secreting cells in the animals of the groups immunized with the formulations with the aggregated protein were similar to those detected in the group that was inoculated with the zika virus (*p*>0.05).

### Example 6. Evaluation of the protective capacity induced in BALB/c mice, by the aggregated-recombinant protein ZC and ZEC formulations.

The protective capacity against a viral challenge in BALB/c mice immunized with the aggregated recombinant proteins ZC and ZEC, was evaluated. Protection was measured as the ability to control or reduce brain viral load in immunized animals after viral challenge with neuroadapted strains.

For the challenge experiment, one month after the last immunization, 10 animals per group were inoculated intracranially with 50 median lethal doses (LD50) of three neuroadapted-different strains (ZIK16 strain, MR766 strain and Brazil ZKV2015 strain) of zika virus. Seven days after the viral challenge, brains of the animals were extracted under aseptic conditions. The brains were macerated, and the supernatants from these samples were used for viral load quantification after the infection on Vero cells.

For the three experiments using different zika viral strains, in animals immunized with the Placebo formulation, a high viral load was observed in the brains samples, greater than 10⁴ PFU/ml. These results are shown in the Figure 9. On the contrary, in the animals of the groups inoculated with the different preparations, which include the recombinant proteins ZC, ZC+ssDNA and ZEC, a reduction in the viral load was observed in comparison to the Placebo group, reaching for some groups statistical significance, depending on the viral strain used (Figure 9A-9C). However, the animals that received the formulation of the recombinant protein ZEC+ssDNA or the zika virus, achieved a significant reduction in viral load, compared to the animals in the Placebo group, consistently in the three challenge trials (*p*<0.001) (Figure 9A-9C). Statistical analysis was performed using an ANOVA test and a Tukey's multiple comparison.

### Example 7. Evaluation of the immunogenicity and the protective capacity of chimeric proteins ZC and ZEC in non-human primates.

Based on the results of preclinical studies in BALB/c mice, the recombinant proteins ZC and ZEC, combined with ssDNA and adjuvanted on alum, were evaluated in zika virusnegative non-human primates. Additionally, a Placebo group was included, which received the same amount of ssDNA as the one used in the aggregation process of the recombinant proteins and alum as adjuvant. Four animals were included in each group of the scheme. Non-human primates were immunized three times with 50 µg or 100 µg doses of the ZC+ssDNA or ZEC+ssDNA aggregate formulations, respectively. The formulations were administered subcutaneously, spaced every two months (day 0, 60 and 120 of the schedule). Blood samples were taken from the animals at the time of each administration (days 0, 60 and 120), and 30 days after each dose (days 30, 90 and 150 of the schedule), to evaluate the induced humoral immune response.

Figure 10A shows the kinetics of the antibody response anti-ZC or ZEC recombinant proteins, measured by ELISA. As can be seen, anti-recombinant protein antibody titers in non-human primates immunized with the ZC+ssDNA and ZEC+ssDNA aggregate formulations begin to be detected 30 days after the first dose, and they increase with the subsequent inoculations. In the animals of the Placebo group, no anti-recombinant protein antibodies were detected in any of the times evaluated.

The antibody response against zika virus was determined by ELISA and Figure 10B shows the kinetics of the titers detected. The antiviral antibody response in non-human primates, immunized with the formulation of ZEC protein+ssDNA, was detected 30 days after the second dose and it was increased with the third administration. On the contrary, in the non-human primates immunized with the formulation of the ZC protein+ssDNA, no antiviral antibodies were detected in any of the times evaluated, like to previous results obtained in mice. This result is mainly due to zika virus capsid protein is enveloped within the native viral particle. Similarly, in the Placebo group, no antiviral antibody titers were detected in any of the times evaluated.

On the other hand, the functionality of the induced antibodies was determined through an *in vitro* viral neutralization test in Vero cells and the zika virus (ZIK16). The results of these determinations are shown in Table 6. In accordance with zika virus antibody response, neutralizing antibody titers were only detected in the group immunized with the aggregated formulation ZEC+ssDNA, 30 days after the second dose, which were boosted after the third administration. Neutralizing antibodies were detected in all the animals that received the ZEC+ssDNA formulation (100% seroconversion), which remained seropositive until day 180 (time of viral challenge). As expected, in the non-human primates immunized with the ZC+ssDNA formulation, no neutralizing antibodies were detected in any of the times evaluated before the viral challenge. This is consistent with the absence of antibody response against the virus observed in this group.

**Table 6. Geometric mean titers of neutralizing antibodies against zika virus in non-human primates immunized with the recombinant chimeric antigen formulations.**

| **Group** | **Immunogen** | **Day 0** | **Day 30** | **Day 60** | **Day 90** | **Day 120** | **Day 150** | **Day 180 (challenge)** |
|---|---|---|---|---|---|---|---|---|
| 1 | Placebo (negative control) | <10 | <10 | <10 | <10 | <10 | <10 | <10 |
| 2 | ZC+ssDNA | <10 | <10 | <10 | <10 | <10 | <10 | <10 |
| 3 | ZEC+ssDNA | <10 | <10 | <10 | 255 | 300 | 655 | 490 |
| % seroconversion | | 0 | 0 | 0 | 100 | 100 | 100 | 100 |

Another parameter measured in the study was the cellular immune response, for that the PBMC were obtained from immunized non-human primates, at three moments of the study: day of the third dose (day 120), one month after the third dose (day 150), and the day of the viral challenge (day 180). Then, the PBMC were cultured *in vitro* and stimulated with the viral antigen (ZIK16 strain), then the number of IFNγ-secreting cells was determined by an ELISpot assay. Figure 11 shows the values obtained at each time tested. As result, IFNγ-secreting cells were detected in all the non-human primates of the groups immunized with the ZC and ZEC recombinant protein formulations, unlike the animals that received the Placebo formulation, where no antiviral cytokine-secreting cells were detected.

To assess the protective capacity against zika virus in immunized animals, all the animals were challenged with the infective virus (ZIK16 strain, 10³ UPF/mL), two months after the last immunization (day 180). The viremia of zika was determined by direct quantification in Vero cells, using the serum of the animals. Figure 12 shows the results obtained which were represented as the mean ± SEM. As can be seen, animals that received the Placebo formulation developed viremia, with a mean of 9 days, and the mean of viral loads were 400 PFU/ml. In the case of the group immunized with the combined formulation ZC+ssDNA, one of the primates was totally protected and the remaining three animals developed viremia, with a mean of 6 days and a lower viral load compared to the Placebo group (with statistical significance). In contrast, animals immunized with the ZEC+ssDNA formulation did not develop viremia and were completely protected (viral load <10 PFU). In general, the chimeric antigens ZC and ZEC, in aggregated form after their combination with ssDNA and alum as adjuvant, induced an immune response capable of controlling zika virus replication in non-human primates.

## Claims

1. Recombinant chimeric antigen that comprises in its polypeptide chain, a polypeptide corresponding to amino acids from 2 to 104 of the capsid protein of zika virus or a polypeptide with an amino acid sequence with almost 90% of identity with the region of the capsid protein of the zika virus.

2. The chimeric antigen according to claim 1, that additionally comprises a polypeptide corresponding to domain III of Envelope protein or a polypeptide with an amino acid sequence with almost 90% of identity with this domain of Envelope protein of zika virus.

3. The chimeric antigen according to claim 1 that additionally comprises a segment of six histidine residues in the N-terminal of its polypeptide chain.

4. The chimeric antigen according to claim 1 that has an amino acid sequence identified as SEQ ID NO: 9 o SEQ ID NO: 10.

5. Vaccine composition that comprises a recombinant chimeric antigen according to claims 1 to 4 and a pharmaceutically approved vaccine adjuvant.

6. Vaccine composition according to claim 5 where the adjuvant is an aluminum salt.

7. Vaccine composition according to claim 5 where the antigen is presented in 10 - 150 micrograms per dose.

8. Vaccine composition according to claim 5 that additionally comprises a nucleic acid that has a nucleotide sequence identified as SEQ ID NO: 11.

9. Use of the recombinant chimeric antigen according to any claims 1 to 4 for the manufacturing of a medicament for the induction of the immune response against zika virus.

10. Method for the induction of the immune response against zika virus in a person wherein is administered a pharmaceutical effective quantity of the recombinant chimeric antigen according to any claims 1 to 4 and a pharmaceutically approved adjuvant.

11. The method of the claim 10 wherein the adjuvant is an aluminum salt.

12. The method of the claim 10 wherein the pharmaceutical effective quantity of the recombinant chimeric antigen is in the range of 10 - 150 micrograms per dose.

13. The method of the claim 10 wherein additionally is administered a nucleic acid that has a nucleotides sequence identified as SEQ ID NO: 11 in combination with the recombinant chimeric antigen.
